# EUROPEAN PATENT SPECIFICATION

(11) **EP 0 402 117 B1**
(45) Date of publication and mention of the grant of the patent: **20.07.1994**
(21) Application number: 90306156.2
(22) Date of filing: 06.06.1990
(51) Int. Cl.: A61M 1/04

(54) **Chest drainage device**
Vorrichtung für die Thoraxdrainage
Dispositif de drainage de la cage thoracique

(30) Priority: 09.06.1989 US 363749; 31.10.1989 US 429892
(43) Date of publication of application: 12.12.1990
(73) Proprietor: SHERWOOD MEDICAL COMPANY, St. Louis, Missouri 63103 (US)
(72) Inventor: Kerwin, Michael John, St. Louis, Missouri 63146 (US); Weilbacher, Eugene Elmer, Ellisville, Missouri 63021 (US); Deweese, Frederick Thane, Ladue, Missouri 63124 (US)
(74) Representative: Porter, Graham Ronald

(56) References cited:
- EP-A- 0 096 579
- US-A- 3 960 165
- US-A- 4 289 158
- US-A- 4 439 190

## Description

This invention relates to chest drainage devices and more particularly to an improved suction control chamber design wherein the overall height of the chest drainage unit may be reduced while allowing the suction control chamber to produce the desired negative pressure in the patient's pleural cavity.

Chest drainage devices for removing fluids from the pleural cavity of a patient generally include a collection chamber, a water seal chamber and a suction control chamber. The suction control chamber operates to limit the negative pressure applied to the collection chamber and the pleural cavity of the patient. During the operation of a chest drainage device, liquid from the patient's pleural cavity is drawn into and accumulated in the collection chamber. Gases are drawn from the pleural cavity of the patient and pass through a water seal in the water seal chamber to the source of suction. The water seal operates as a barrier to prevent the patient's pleural cavity from being exposed to the atmosphere and also prevents the patient's pleural cavity from being in direct flow of communication with the vacuum source.

U.S. Patent No. 3,783,870, issued to Schachet on January 8, 1974, and U.S. Patent No. 4,439,190, issued to Protzmann et al on March 27, 1984 describe the operation of a typical chest drainage device. US-A-4289158 discloses a chest drainage collection system including a manifold to which a suction control container, a water seal container, and one or more collection containers are releasably secured. The manifold includes a first passage communicating between the suction control and water seal containers and a second passage communicating between the water seal and collection containers. The system includes a suction control apparatus including means for reducing the loss of liquid in the suction control container due to entrainment in air and means for attenuating noise in the suction control container. A flexible plastic sleeve is provided for transmitting atmospheric air into an air chamber above the liquid in the suction control container without contact with the liquid in that container during the transmission.

The present invention is readily adaptable for use in an integral one-piece chest drainage device or a multi-bottle chest drainage device similar to the chest drainage devices referenced above. Generally, the suction control chamber allows the user to apply a prescribed vacuum pressure to the pleural cavity of a patient by adding a predetermined amount of liquid to the suction control chamber. The commonly used chest drainage device utilizes a suction control chamber which is basically an unequal-legged water manometer to regulate the suction pressure being applied to the pleural cavity of a patient. This type of suction control chamber generally consists of a pair of legs or columns interconnected at their bottom ends. The top of the generally smaller, first column, is open to the atmosphere. The second column is generally larger than the first column and includes a top end in flow communication with the vacuum source and the pleural cavity of the patient.

The overall height of the suction control chamber typically dictates the minimum height of the chest drainage device. Commonly available chest drainage devices have an overall height of approximately 40 cm. In the typical suction control chamber, approximately 25 cm. is attributable to the operational range of the chest drainage device. The remaining height of the chest drainage device is attributable to the air/water separation space located above the water fill level and the height of the base or stand of the chest drainage device.

The effectiveness of the air/water separation space at any given air flow rate is determined by the overall suction control chamber geometry and the height of the suction control chamber above the liquid level. If the air flow rate through the suction control chamber is too high, liquid is entrained in the air and will be carried out of the suction control chamber. If this occurs, the vacuum pressure being applied to pleural cavity of the patient will gradually decrease as the liquid level in the suction control chamber is depleted. Additionally, the liquid from the suction control chamber may contaminate the vacuum source and/or be deposited within other chambers of the chest drainage device. Certain chest drainage devices have incorporated baffles in the top of the suction control chamber in an effort to decrease the required height of the air/water separation space and to prevent the loss of water in the suction control chamber.

In a chest drainage device, the pressure applied to the pleural cavity of the patient is dependent on the dynamic water height of the liquid in the suction control chamber. For example, if the desired patient pressure is 20 cm. H₂O of vacuum pressure, a dynamic water height of at least 20 cm. is required in the suction control chamber. In chest drainage devices which utilize a water seal chamber, the water seal chamber will typically add approximately 2 cm. H₂O of resistance so that if the desired patient pressure is 20 cm. H₂O, the suction control chamber must provide 22 cm. H₂O of vacuum pressure to overcome the resistance of the water seal chamber. The standard operating ranges for most chest drainage devices is between 5 and 25 cm. H₂O vacuum pressure. Therefore, in order to have the capability of supplying 25 cm. H₂O of vacuum pressure to the pleural cavity of the patient, this portion of the suction control chamber must be at least 25 cm. high. The baffle systems used in certain chest drainage devices are designed to prevent liquid from being entrained in the air and may be used to reduce the height of the air/water separation space and do not materially effect the dynamic water height of the suction control chamber. Therefore, unless valves or other flow restricting devices are used, the height of a chest drainage device must be at least 25 cm. plus the height of the air/water space or baffle chamber and the base of the chest drainage device.

### Summary of the Invention

The invention provides a chest drainage device for removing fluids and gases from the body of a patient comprising
a collection chamber for flow communication with the body of a patient wherein fluid from the body of a patient will be collected therein,
said collection chamber being adapted for communication with a source of vacuum pressure to apply a desired vacuum pressure to the body of a patient to draw fluids into said collection chamber,
a liquid containing suction control chamber for flow communication with the atmosphere and said source of vacuum pressure,
said suction control chamber having first and second columns formed therein and said first and second columns having top ends and bottom ends and wherein said bottom ends are in flow communication with each other and said top end of said first column is in flow communcation with the atmosphere and said top end of said column is in flow communication with said source of vacuum pressure when the device is in use, characterised in that the control chamber includes
a float means in flow communication with said first column wherein in use said float means selectively restricts the flow of fluid between the source of vacuum pressure and the atmosphere to regulate the amount of vacuum pressure applied to the body of a patient by said source of vacuum pressure.

The invention also includes a suction control chamber assembly comprising the control chamber as defined above.

In accordance with one form of the present invention, the improved chest drainage device is a type of chest drainage device typically known as a three-bottle chest drainage device. Thus, the improved chest drainage device of the present invention preferably includes a collection chamber adapted to be in fluid communication with the patient's pleural cavity; a water seal chamber in flow communication with the collection chamber; and an improved suction control chamber. Additionally, the illustrated form of the present invention includes a manifold which maintains flow communication among the various chambers of the chest drainage device.

The suction control chamber of the present invention is comprised of a pair of generally elongate columns in fluid communication at their bottom ends. The smaller, first column extends downwardly into the larger, second column. The top end of the first column is open to the atmosphere while the bottom end of the first column includes a moveable float chamber generally oriented in flow communication with the bottom opening of the first column. Preferably, an elongated sleeve is provided attached to the top of the float chamber to movably surround the first column.

In the preferred form of the present invention, the second column is larger than the first column and substantially encloses the first column. The top end of the second column is in flow communication with the vacuum source and the pleural cavity of the patient. The height of the second column is generally divisible into two sections, the first section is the air/water separation space and is located near the top end of the second column. The second section is the suction control section which extends from the bottom end of the suction control chamber upwardly to the air/water separation space. The suction control section is the portion of the suction control chamber which is typically pre-filled with liquid to create the dynamic water height which controls the amount of vacuum pressure actually being applied to the pleural cavity of the patient. In the present invention, this liquid is partially replaced by the float chamber so that the desired dynamic water height may be obtained without using the amount of liquid required in previous chest drainage devices.

An advantage of the present invention is that the improved suction control chamber allows for the construction of a versatile chest drainage device which has either a smaller overall height than the presently available chest drainage devices or has a greater operational range than presently available chest drainage devices without increasing the height of the chest drainage device.

Another advantage of the present invention is that the operational range of the chest drainage device is linearly adjustable by adding or removing incremental amount of liquid from the suction control chamber.

Further advantage of the improved suction control chamber is that it is readily adaptable for use on nearly any chest drainage device.

These, as well as other features and advantages of the present invention will become apparent from the following detailed description and accompanying drawings.

### Brief Description of the Drawings

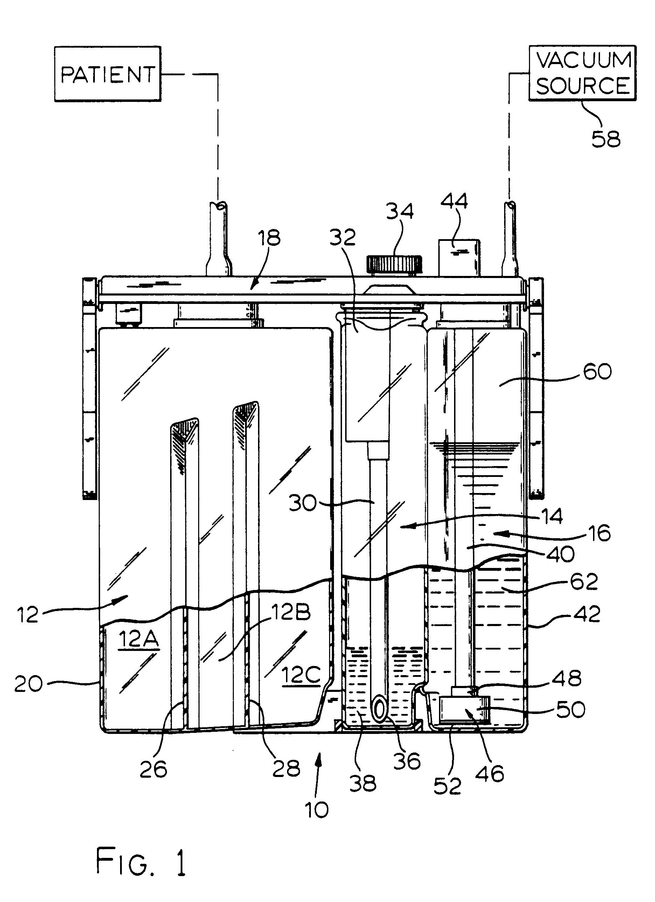

Fig. 1 is a cross-sectional side view of the preferred embodiment of the present invention;
Fig. 2 is an enlarged cross-sectional view of an alternative embodiment of the suction control chamber of the present invention;
Fig. 3 is a cross-sectional view taken along lines 3--3 of Fig. 2; and
Fig. 4 is a perspective view of the float chamber of the present invention.
Fig. 5 is a cross-sectional side view of an alternative embodiment of the present invention;
Fig. 6 is an enlarged cross-sectional view of the suction control chamber of the present invention;
Fig. 7 is a cross-sectional view of an alternative embodiment of the present invention taken along lines 7--7 of Fig. 8; and
Fig. 8 is an enlarged cross-sectional view of the suction control chamber of the alternative embodiment of the present invention illustrated in Fig. 7.

### Detailed Description of the Preferred Embodiment

A preferred design of the present invention is illustrated in the attached drawings and is designated herein, generally, as chest drainage device 10. The chest drainage device of the present invention consists primarily of a collection chamber 12, water seal chamber 14, a suction control chamber 16 and a manifold 18. In the preferred embodiment, the manifold 18 is attached to the top of the collection chamber 12 and the suction control chamber 16 is attached to the manifold 18 adjacent to the water seal chamber 14. The manifold 18 provides the desired flow communication between the collection chamber 12, the water seal chamber 14 and the suction control chamber 16, thus, eliminating the need for hoses, conduits and the like frequently used in a variety of other chest drainage devices.

In order to provide for the accurate measurement of the fluid collected from the pleural cavity of the patient, the collection chamber is provided with inner walls 26 and 28, which, in combination with the outer walls 20, divide the collection chamber 12 into three compartments, 12A, 12B, and 12C. The height of wall 26 is less than the wall 28 so that once compartment 12A is filled, fluid will flow into 12B, after compartment 12B is filled, fluid will flow into spill over into compartment 12C. Each compartment has graduations thereon so that the attending physician or nurse can readily determine the amount of drainage collected from the pleural cavity of the patient.

The water seal chamber 14 includes a water seal column 30 and a baffle chamber designated generally as 32. Liquid is poured into the water seal chamber 14 through the fill cap 34 until it reaches the fill line indicated on the side of the water seal chamber 14. The volume of liquid placed in the water seal chamber 14 should be sufficient to continuously cover the bottom opening 36 of the water seal column 30 during the typically operation of the chest drainage device 10. This volume of liquid is commonly known as a water seal and is referred herein as the water seal 38.

The suction control chamber 16 includes a generally cylindrical first column 40 which is surrounded and substantially enclosed by a larger second column 42. The top end of the first column 40 includes a fill opening 44 which is open to the atmosphere and allows liquid to be poured into the suction control chamber 16. The first column 40 extends downwardly into the suction control chamber 16 from the fill opening 44 to a float chamber 46 which movably contacts the open bottom end of the first column 40. The float chamber 46 consists of a retaining ridge 48 on the top surface thereof; a body section 50 and a lower base section 52. The body section 50 includes a centrally positioned recess 56 which slidably receives the bottom end of the first column 40. The inner diameter of the recess 56 is slightly larger than the outer diameter of the first column to allow air to pass therebetween. The retaining ridge 48 extends upwardly from the body section 50 to further retain the first column 40 therein and to maintain the float chamber 46 in a slidable, generally contacting relation with the bottom end of the first column. The body section 50 and the base section 52 cooperate to create a float chamber 46 having a precisely defined buoyancy.

The second column 42 of this embodiment includes a top end in flow communication with the vacuum source 58 and the pleural cavity of the patient through a variety of passageways in the manifold 18. The bottom end of the second column 42 is in flow communication with the bottom end of the first column 40 and the float chamber 46. The second column 42 is generally divisible into two sections; the top section is the air/water separation space 60 and the lower, second section, is the suction control section 62. The air/water separation space 60 operates to prevent liquid from being drawn into the vacuum source 58 or into the collection chamber 12 of the chest drainage device 10. The height of the air/water space 60 in the preferred embodiment, is typically 10-17 cm. high and may be reduced through the use of a variety of baffles (not shown) or by modifying the overall geometry of the suction control chamber 16.

The lower section of the second column 42 is the suction control section 62. This section of the second column 42 creates the dynamic water height of the liquid in the suction control chamber 16 and determines the amount of vacuum pressure actually applied to the pleural cavity of the patient. The typical chest drainage device applies between 5 and 25 cm. H₂O to the pleural cavity of the patient and therefore the minimum height of the suction control section 62 is typically 25 cm. In the present invention, when liquid is added to the suction control chamber 16, the volume and buoyancy of the float chamber 46 decreases the amount of liquid necessary to create the desired dynamic water height and therefore the overall height of the suction control section may be reduced to produce the desired patient vacuum pressure.

For example, in the typical suction control chamber 16, liquid is added to the bottom of the suction control chamber 16 to a desired level corresponding to the amount of vacuum pressure to be applied to the pleural cavity of the patient. This is commonly known as the dynamic water height of the suction control chamber 16. At this level, the hydrostatic pressures in the second column 42 cause a certain amount of liquid to flow upwardly into the bottom of the first column 40 until the upward pressure is balanced by the downward pressure in the first column 40. This downward pressure is caused by the atmospheric pressure which enters the first column through the top fill opening 44. During operation of the chest drainage device 10, the vacuum source 58 applies a vacuum pressure to the top of the second column 42 and causes the liquid to be drawn from the first column 40 into the bottom section of the second column 42. As this occurs, air is pushed by atmospheric pressure into the suction control chamber 16 through the first column 40 and into the second column 42 to offset the pressure difference between the dynamic water height of the column and the vacuum pressure being applied by the vacuum source 58.

The float chamber 46 of the present invention normally operates to apply a buoyant force to the bottom opening of the first column 40. This buoyant force is directed upwardly against the bottom of the first column 40 and provides an added second force in addition to the dynamic water height of the suction control chamber 16 that must be overcome before air is pushed into the suction control chamber 16. When the vacuum source 58 applies a vacuum pressure in excess of the dynamic water height of the suction control chamber 16 and the buoyant force of the float chamber 46, air is pushed into the suction control chamber 16 through the first column 40 to offset the difference. Therefore, if the liquid in the suction control chamber 16 provides a dynamic water height of approximately 15 cm. H₂O and the float chamber 46 applies a buoyancy force equivalent to 5 cm. a vacuum pressure in excess of 20 cm. H₂O will be required before air is pushed into the suction control chamber 16 through the first column 40 by the atmosphere. Therefore, by decreasing the required dynamic water height of the suction control chamber 16 by 5 cm. the overall height of the suction control chamber 16 and chest drainage device 10 may also be reduced by approximately 5 cm.

This capability is also useful where it is desirable to increase the operating range of the suction control chamber 16 without increasing the overall height of the suction control chamber 16. In the past, it was necessary to add a separate vacuum regulator and bypass the suction control chamber 16 to obtain higher vacuum pressures. With the present invention, it is possible to design a suction control chamber 16 capable of applying higher vacuum pressures to the pleural cavity of the patient without significantly increasing the overall height of the suction control chamber 16 by using a float chamber of the type described herein.

In the second embodiment illustrated in Fig. 2, a damper 80 is used to create a substantially closed damper chamber 84 adjacent to the body section 50 of the float chamber 46. The damper 80 of this embodiment consists of an inwardly directed ridge in the bottom of the suction control chamber 16 which extends inwardly from the side of the suction control chamber to a location adjacent in the float chamber 46. The use of the damper 80 will decrease pressure fluctuations caused by erratic movement between the float chamber 46 and the bottom of the first column 40. When the float chamber 46 moves downwardly from the bottom of the first column 40, liquid in the damper chamber 84 must be displaced to allow for the downward movement of the float chamber 48. In order for this to occur, liquid in the damper chamber 84 must flow around the sides of the float chamber 46 and past the damper 80. Likewise, when the float chamber 46 moves upwardly, liquid must flow downwardly into the area of the damper chamber 84 vacated by the float chamber 46.

The chest drainage device 10 of the present invention functions similar to other chest drainage devices and is briefly discussed herein to assist in describing the overall operation of the present chest drainage device 10. Operation of the chest drainage device 10 of the present invention includes the preliminary steps of adding a predetermined amount of liquid to the water seal chamber 14 to create the water seal 38 and adding a predetermined amount of liquid to the suction control section 62 of the suction control chamber 16 to a level sufficient to provide the desired patient vacuum pressure. Once the vacuum hose is attached to the vacuum source 58 and the drainage tube is attached to the patient's pleural cavity, the process of suctioning fluid from the patient's pleural cavity is begun. If the vacuum pressure from the vacuum source 58 exceeds the prescribed patient vacuum level, atmospheric air is pushed into the suction control chamber 16 through the fill opening 44; into the first column 40 and past the float chamber 46 into the second column 42 to decrease the vacuum pressure actually applied to the pleural cavity of the patient.

During normal operation of the chest drainage device 10, the water seal 38 in the water seal chamber 14 may be drawn upwardly in the water seal column whenever the patient inspires (negative pressure). The liquid will flow downwardly in the water seal column 30 and into the reservoir area 33 of the water seal chamber 14 whenever the patient breathes out (positive pressure). As the fluid is drawn from the patient's pleural cavity, the liquid is collected in the collection chamber 12 and any gases drawn from the pleural cavity of the patient will flow through the water seal chamber 14 and into the vacuum source 58. As described previously, the operation of the water seal chamber adds approximately 2 cm. H₂O of resistance so that the typical suction control chamber 16 must provide approximately 22 cm. H₂O of vacuum pressure to overcome the additional resistance created by the operation of the water seal chamber 14.

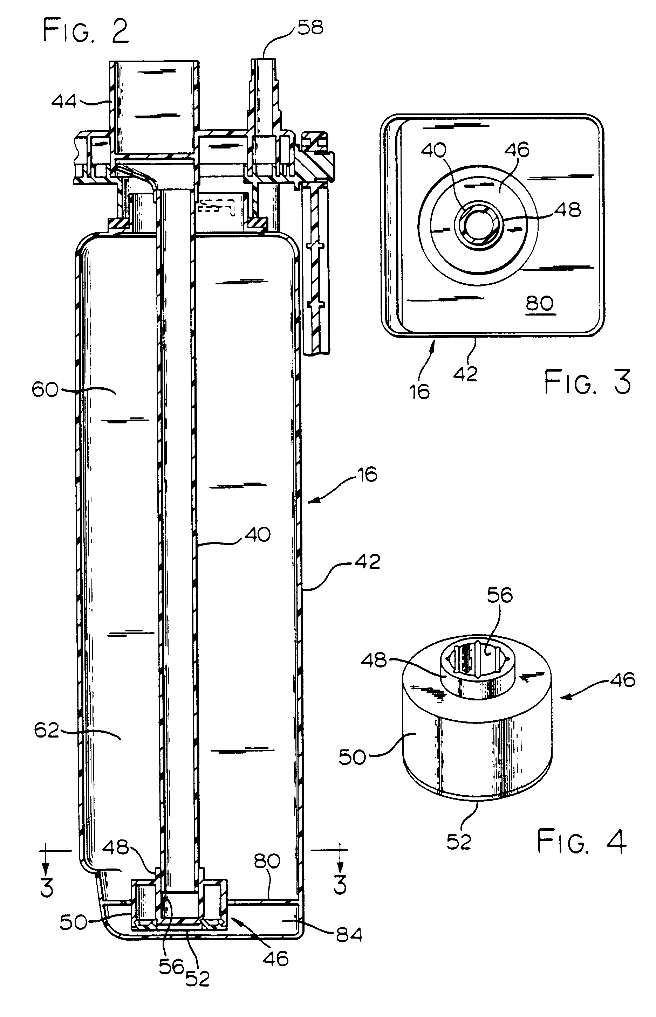

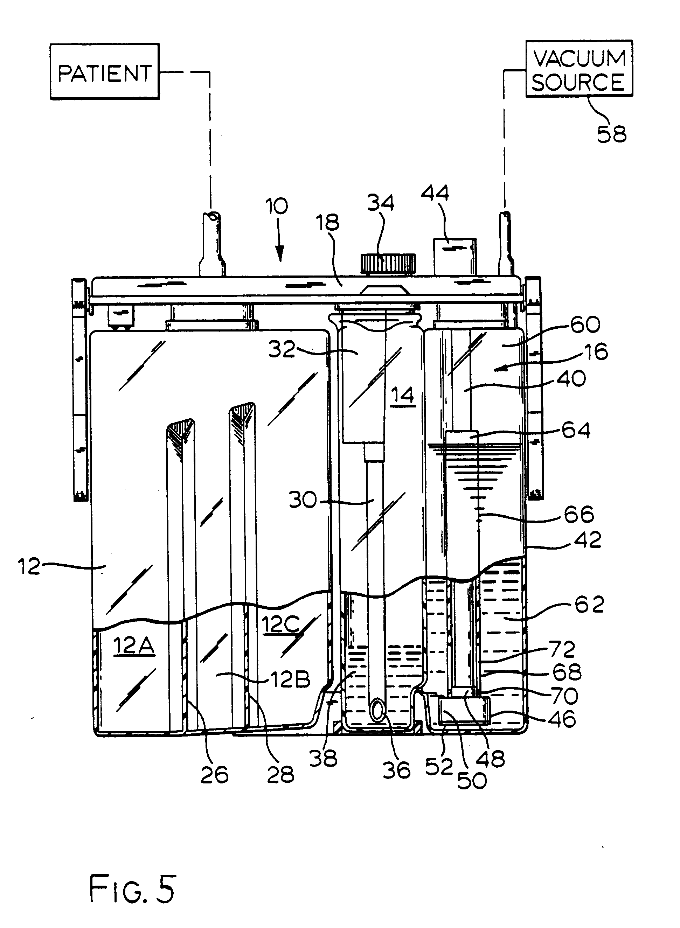

A third embodiment of the present invention is illustrated in Figs. 4-8. Common numbers have been assigned to elements which are common to the above-described elements. As described above, the chest drainage device 10 of the present embodiment consists generally of a collection chamber 12, a water seal chamber 14, a suction control chamber 16 and a manifold 18. The manifold 18 is attached to the top of the collection chamber 12 and the suction control chamber 16 is attached to the manifold 18 adjacent to the water seal chamber 14. The manifold 18 provides the desired flow communication between the collection chamber 12, the water seal chamber 14 and the suction control chamber 16, thus, eliminating the need for hoses, conduits and the like frequently used in a variety of other chest drainage devices.

The suction control chamber 16 of the present embodiment includes a generally elongate cylindrical first column 40 which is surrounded and substantially enclosed by a larger second column 42. The top end of the first column 40 includes a fill opening 44 which is open to the atmosphere and allows liquid to be readily added to the suction control chamber 16. The first column 40 extends downwardly into the suction control chamber 16 from the fill opening 44 to a float chamber 46 which movably contacts the open bottom end of the first column 40. A ballast sleeve 64 is attached to the top of the float chamber 46 and extends upwardly along the first column 40.

As in the first and second embodiments, the float chamber 46 consists of a retaining ridge 48 on the top surface thereof; a body section 50 and a lower base section 52. The body section 50 includes a centrally positioned recess 56 which slidably receives the bottom end of the first column 40. The inner diameter of the recess 56 is slightly larger than the outer diameter of the first column 40 to allow air to pass therebetween. The retaining ridge 48 extends upwardly from the body section 50 to further retain the first column 40 therein and to maintain the float chamber 46 in a slidable, generally contacting relation with the bottom end of the first column 40. The body section 50 and the base section 52 cooperate to create a float chamber 46 having a precisely defined buoyancy by retaining a specific amount of air therein.

In the present embodiment, when liquid is added to the suction control chamber 16, the volume and buoyancy of the float chamber 46 decreases the amount of liquid necessary to create the desired dynamic water height and therefore reduces the overall height of the suction control section 62. The addition of the ballast sleeve 64 to the float chamber 46 allows the patient suction pressure to be adjusted linearly so that the patient vacuum pressure may be increased or decreased by adding or removing an incremental amount of liquid to the suction control chamber 16.

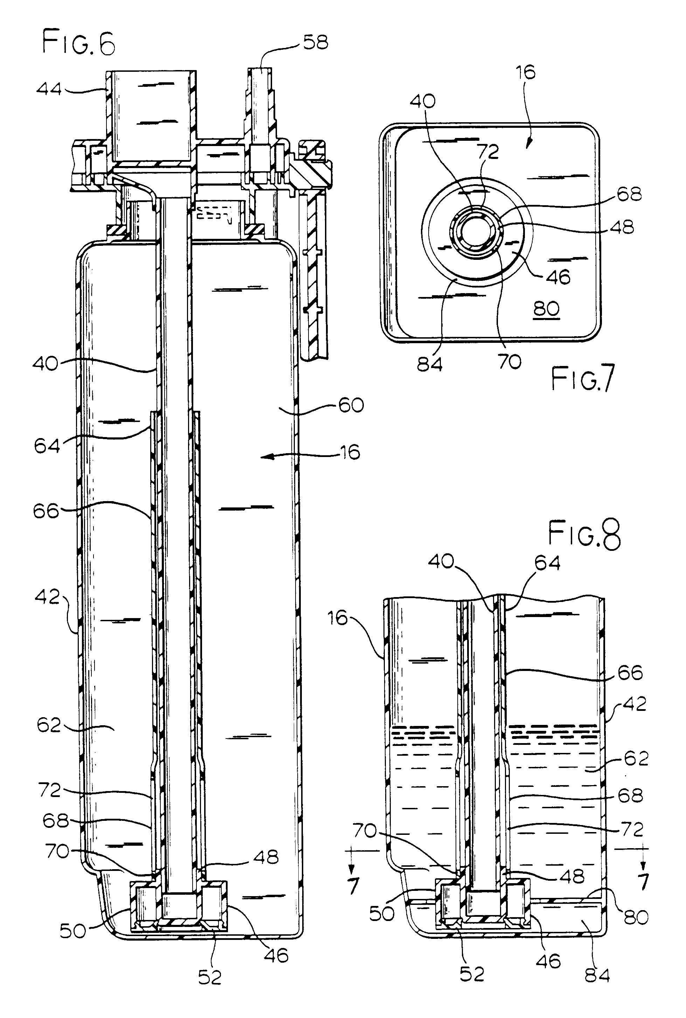

As best illustrated in Figs. 6 and 8, a ballast sleeve 64 is added to the float chamber 46 so that the pressure applied to the pleural cavity of the patient varies linearly during the operational range of the chest drainage device 10. The ballast sleeve 64 consists generally of a top, first section 66 and a lower, second section 68. The top section 66 is generally cylindrical and is designed to slidably surround nearly the entire length of the first column 40 without causing excess frictional resistance therebetween. The lower section 68 extends downwardly from the top section 66 and tapers outwardly to a ballast collar 70 which engages the retaining ridge 48 on the float chamber 46. The lower section 68 includes a pair of tapered openings 72 which are surrounded by a pair of extensions 74 that allow atmospheric air to pass from the bottom of the first column 40 and into the second column 42 through the tapered openings 72. The addition of the-ballast sleeve 64 to the float chamber 46 allows the buoyancy pressure created by the relationship between the float chamber 46 and the ballast sleeve 64 to increase linearly with increases in the fluid level in the suction control chamber 16 and also allows the chest drainage device to operate at a lower initial vacuum pressure than is possible with the float chamber 46 alone.

Fig. 8 also illustrates the use of the damper 80, as described previously, to create a substantially closed damper chamber 84 adjacent to the body section 50 of the float chamber 46. The use of the damper 80 will decrease pressure fluctuations caused by erratic movement between the float chamber 46 and the bottom of the first column 40. When the float chamber 46 moves downwardly from the bottom of the first column 40 to allow air to flow through the first column 40, liquid in the damper chamber 84 must be displaced to allow for the downward movement of the float chamber 46. In order for this to occur, liquid in the damper chamber 84 must flow around the sides of the float chamber 46 and past the damper 80. Likewise, when the float chamber 46 moves upwardly to prevent air from flowing through the first column 40, liquid must flow downwardly and into the area of the damper chamber 84 vacated by the float chamber 46.

As an example of the operation of the present embodiment, a dynamic water height of 7 cm. will produce a suction control chamber pressure of approximately 7 cm. H₂O to create a patient vacuum pressure of approximately 5 cm. H₂O when a water seal chamber 14 is used. At a dynamic water height of approximately 17 cm., a suction control chamber pressure of approximately 27 cm. H₂O will be created to apply a vacuum pressure of approximately 25 cm. H₂O to the pleural cavity of the patient when a water seal chamber 14 is used.

An important factor in the operation of the present invention is the portion of the ballast sleeve 64 which is present above the liquid level at any given time. The density of the ballast sleeve 64 is preferably at or near the density of water so that the submerged portion of the ballast sleeve 64 will not materially affect the buoyancy pressure created by the float chamber 46. The portion of the ballast sleeve 64 above the liquid level in the suction control chamber 16 will exert a downward pressure on the float chamber 46 to oppose the buoyancy pressure created by the submerged float chamber 46. Therefore, when the dynamic water height in the suction control chamber 16 is relatively small, the portion of the ballast sleeve 64 above the liquid level will exert a greater downward pressure to oppose the buoyancy pressure created by the float chamber 46. As the dynamic water height is increased, less of the ballast sleeve 64 will be above the liquid level and the ballast sleeve 64 will exert a smaller downward pressure against the buoyancy pressure of the float chamber 46. This relationship between the portion of the ballast sleeve 64 above the liquid level and the buoyancy pressure created by the float chamber 46 provides an overall operational pressure in the suction control chamber 16 which is within the typical operating ranges of a chest drainage device 10 while allowing the overall height of the suction control chamber 16 to be reduced.

By maintaining the preferred density of the ballast sleeve 64 at or near the density of water, the relationship between the portion of the ballast sleeve 64 above the liquid level and the buoyancy pressure of the float chamber 46 also creates a suction control chamber 16 wherein a visually readable linear scale may be used on the side of the suction control chamber 16 so that the user may easily modify the desired patient vacuum pressure by adding or removing incremental amounts of liquid from the suction control chamber 16. If the density of the ballast sleeve 64 is decreased, the float chamber 46 would operate within a limited range of dynamic water heights and the operational range of the chest drainage device 10 would be reduced. A chest drainage device 10 of this type would be able to produce the desired vacuum pressures within a limited operational range such as between 15 and 20 cm. H₂O. If the density of the ballast sleeve 64 is increased, the float chamber 46 would operate over a larger range of dynamic water heights and therefore, the operational range of the chest drainage device 10 would be increased; however, the scale along the side of the suction control chamber 16 would be compressed such that it would be very difficult to accurately adjust the suction control chamber to provide the desired vacuum pressure to the patient's pleural cavity. Any small change in the amount of liquid in the suction control chamber 16 would dramatically alter the vacuum pressure applied to the patient.

While the preferred form of the invention has been described with reference to one specific type of drainage device, it will be apparent that various changes and modifications thereto may be made without departing from the true scope of the invention as defined by the following claims. For example, the general shape or design of the float chamber 46 or ballast sleeve 64 may be readily modified to be adaptable for use with nearly any suction control chamber design as long as the float chamber is able to apply a buoyancy pressure against the equivalent of a first column and is moveable to allow air or liquid to flow between the respective columns. Additionally, it is readily anticipated that the suction control chamber of the present invention may be an integral part of a chest drainage device or a separate suction control chamber which may be attached to nearly any chest drainage device without substantial modification or alteration of the present invention.

## Claims

1. A chest drainage device (10) for removing fluids and gases from the body of a patient comprising
a collection chamber (12) for flow communication with the body of a patient wherein fluid from the body of a patient will be collected therein,
said collection chamber being adapted for communication with a source of vacuum pressure (58) to apply a desired vacuum pressure to the body of a patient to draw fluids into said collection chamber,
a liquid containing suction control chamber (16) for flow communication with the atmosphere and said source of vacuum pressure (58),
said suction control chamber (16) having first and second columns (40, 42) formed therein and said first and second columns having top ends and bottom ends and wherein said bottom ends are in flow communication with each other and said top end of said first column is in flow communication with the atmosphere and said top end of said second column is in flow communication with said source of vacuum pressure (58), when the device is in use, characterised in that the control chamber includes
a float means (46) in flow communication with said first column (40) wherein in use said float means selectively restricts the flow of fluid between the source of vacuum pressure (58) and the atmosphere to regulate the amount of vacuum pressure applied to the body of a patient by said source of vacuum pressure (58).

2. The device of Claim 1, wherein said float means comprises a buoyant float member (46) operationally positioned in the liquid contained in said suction control chamber (16) and in flow communication with said bottom end of said first column (40).

3. The device of Claim 1 or Claim 2, wherein said suction control chamber (16) further includes a damper member (80) extending inwardly into said suction control chamber (16) to a position adjacent to said float member (46) to form a lower chamber section (84) to inhibit the flow of liquid in said suction control chamber (16).

4. The device of Claim 1, Claim 2 or Claim 3, wherein the float means (46) allows atmospheric air to be pushed into the suction control chamber (16) through the first column (40) when the vacuum pressure applied by the source of vacuum pressure (58) is greater than the desired patient vacuum pressure.

5. The device of any one of the preceding Claims, wherein the liquid in the suction control chamber (16) forms a dynamic water height between the first and second columns (40, 42) to resist the flow of air from the atmosphere into the suction control chamber (16) and the float means (46) provides an additive buoyant force to resist the flow of air from the atmosphere into the suction control chamber (16).

6. A suction control chamber assembly (16) in flow communication with a collection chamber of a drainage device for removing fluids from the body of a patient, wherein the suction control chamber assembly comprises,
a first column (40) having top and bottom ends wherein said top end is in flow communication with the atmosphere,
a second column (42) having top and bottom ends wherein said bottom end is in flow communication with said first column and said top end is in flow communication with a source of vacuum pressure (58), characterised in that the assembly includes
a float member (46) buoyantly in flow communication with said first column (40) to selectively restrict flow communication between the source of vacuum pressure (58) and the atmosphere to regulate the amount of vacuum pressure applied to the body of a patient

7. The suction control chamber of Claim 6, wherein said suction control chamber (16) further includes a damper (80) in the bottom end of said suction control chamber (16) to form a liquid flow restricting chamber therein.

8. A chest drainage device as claimed in any one of Claims 1 - 5, characterised in that the control chamber (16) includes a ballast means (64) extending upwardly from said float means (46) within said second column (42).

9. The device of Claim 8, wherein the float means (46) comprises a float chamber (46) in flow communication with the bottom end of said first column (40).

10. The device of Claim 8 or Claim 9, wherein the ballast means (64) comprises a ballast sleeve (64) substantially surrounding said first column (40).

11. The device of Claim 8, 9 or 10, wherein the ballast means (64) has a density approximately equal to the density of the liquid in said suction control chamber (16).

12. The device of Claim 11, wherein the float means comprises a float chamber (46) which selectively blocks the bottom end of said first column (40) in response to the difference between the vacuum pressure applied by the source of vacuum pressure (58) to the float means and atmospheric pressure.

13. The device of Claim 12, wherein the float means allows atmospheric air to be pushed into the suction control chamber through the first column (40) when the vacuum pressure applied by the source of vacuum pressure (58) is greater than the desired patient vacuum pressure.

14. The suction control chamber of Claim 10, wherein the float means includes a buoyant float chamber (46) and the ballast means (64) comprises a ballast sleeve (64) and wherein the ballast sleeve extends upwardly from the float chamber (46) to movably surround part of the first column (40).

15. The suction control chamber of Claim 14 wherein the ballast sleeve (64) includes an upper (66) and a lower section (68) wherein the lower section (68) includes an opening therein to allow flow communication therethrough between the first and second columns (40, 42).

16. A device as claimed in any one of of Claims 1 - 5, wherein the second column (42) is larger than the first column (40) and substantially encloses the first column (40).

## Patentansprüche

1. Vorrichtung (10) zur Toraxdrainage zum Entfernen von Flüssigkeiten und Gasen aus dem Körper eines Patienten, umfassend
eine Sammelkammer (12) zur Fließkommunikation mit dem Körper eines Patienten, in der ein Fluid aus dem Körper eines Patienten gesammelt wird,
wobei die Sammelkammer für die Kommunikation mit einer Unterdruckquelle (58) eingerichtet ist, um einen gewünschten unterdruck an den Körper eines Patienten anzulegen, um Fluide in die Sammelkammer abzusaugen,
eine Flüssigkeit enthaltende Ansaug-Kontrollkammer (16) zur Fließkommunikation mit der Atmosphäre und der Unterdruckquelle (58),
wobei diese Ansaug-Kontrollkammer (16) eine erste und zweite Säule (40,42) aufweist und die erste und zweite Säule obere und untere Enden aufweisen und wobei die unteren Enden in Fließkommunikation miteinander stehen und das obere Ende der ersten Säule in Fließkommunikation mit der Atmosphäre steht und das obere Ende der zweiten Säule in Fließkommunikation mit der Unterdruckquelle (58) steht, wenn die Vorrichtung im Gebrauch ist,
dadurch gekennzeichnet, daß
die Kontrollkammer ein Auftriebsmittel (46) in Fließkommunikation mit der ersten Säule (40) aufweist, wobei das Auftriebsmittel beim Gebrauch den Durchfluß des Fluids zwischen der Unterdruckquelle (58) und der Atmosphäre selektiv einschränkt, um den Betrag an Unterdruck, der durch die Unterdruckquelle (58) an den Körper eines Patienten angelegt wird, zu regulieren.

2. Vorrichtung gemäß Anspruch 1, worin das Auftriebsmittel einen schwimmfähigen Auftriebskörper (46) umfaßt, der funktionsgemäß in der Flüssigkeit angeordnet ist, die sich in der Ansaug-Kontrollkammer (16) befindet, und die in Fließkommunikation mit dem unteren Ende der ersten Säule (40) steht.

3. Vorrichtung gemäß Anspruch 1 oder Anspruch 2, worin die Ansaug-Kontrollkammer (16) zusätzlich eine Dämpfungseinrichtung (80) aufweist, die sich einwärts in die Ansaug-Kontrollkammer (16), bis zu einer, an den Auftriebskörper (46) grenzenden Stelle, erstreckt, so daß ein unterer Kammerabschnitt (84) gebildet wird, der den Flüssigkeitsstrom in der Ansaug-Kontrollkammer (16) hemmt.

4. Vorrichtung gemäß Anspruch 1, 2 oder 3, worin das Auftriebsmittel (46) ermöglicht, daß Umgebungsluft durch die erste Säule (40) in die Ansaug-Kontrollkammer (16) eingelassen wird, sobald der Unterdruck, der von der Unterdruckquelle (58) angelegt wird, größer ist als der gewünschte Unterdruck an dem Patienten.

5. Vorrichtung nach einem der vorangegangenen Ansprüche, worin die Flüssigkeit in der Ansaug-Kontrollkammer (16) einen dynamischen Überstand zwischen der ersten und der zweiten Säule (40,42) erzeugt, so daß Widerstand gegen das Einströmen von Luft aus der Atmosphäre in die Ansaug-Kontrollkammer (16) geleistet wird, und worin das Auftriebsmittel (46) eine zusätzliche Auftriebskraft zur Verfügung stellt, um dem Einströmen von Umgebungsluft in die Ansaug-Kontrollkammer (16) entgegenzuwirken.

6. Ansaug-Kontrollkammer (16) in Fließkommunikation mit einer Sammelkammer einer Drainagevorrichtung, zur Entfernung von Fluiden aus dem Körper eines Patienten, wobei die Ansaug-Kontrollkammer umfaßt,
eine erste Säule (40) mit oberem und unterem Ende, wobei das obere Ende in Fließkommunikation mit der Atmosphäre steht,
eine zweite Säule (42) mit oberem und unterem Ende, wobei das untere Ende in Fließkommunikation mit der ersten Säule steht und das obere Ende in Fließkommunikation mit einer Unterdruckquelle (58) steht,
dadurch gekennzeichnet, daß
die Anordnung einen Auftriebskörper (46) umfaßt, der schwimmfähig in Fließkommunikation mit der ersten Säule (40) steht, so daß die Fließkommunikation zwischen der Unterdruckquelle (58) und der Atmosphäre selektiv eingeschränkt wird, um den Betrag des Unterdrucks, der am Körper eines Patienten angelegt wird, zu regulieren.

7. Ansaug-Kontrollkammer gemäß Anspruch 6, wobei die Ansaug-Kontrollkammer (16) ferner am unteren Ende der Ansaug-Kontrollkammer (16) einen Dämpfer (80) umfaßt, so daß eine Kammer gebildet wird, die den Flüssigkeitsstrom einschränkt.

8. Vorrichtung zur Toraxdrainage gemäß einem der Ansprüche 1 - 5, dadurch gekennzeichnet, daß die Kontrollkammer (16) ein Ballastmittel (64) umfaßt, das sich von dem Auftriebsmittel (46) aus, innerhalb der zweiten Säule (42), nach oben erstreckt.

9. Vorrichtung gemäß Anspruch 8, wobei das Auftriebsmittel (46) eine Auftriebskammer (46) umfaßt, die in Fließkommunikation mit dem unteren Ende der ersten Säule (40) steht.

10. Vorrichtung gemäß Anspruch 8 oder 9, wobei das Ballastmittel (64) eine Ballasthülse (64) umfaßt, die einen beträchtlichen Teil der ersten Säule (40) umgibt.

11. Vorrichtung gemäß Anspruch 8, 9 oder 10, wobei das Ballastmittel (64) eine etwa gleiche Dichte wie die Dichte der Flüssigkeit in der Ansaug-Kontrollkammer (16) besitzt.

12. Vorrichtung gemäß Anspruch 11, worin das Auftriebsmittel eine Auftriebskammer (46) umfaßt, die selektiv das untere Ende der ersten Säule (40), in Abhängigkeit vom Druckunterschied zwischen dem unterdruck, der von der Unterdruckquelle (58) auf das Auftriebsmittel ausgeübt wird, und dem Atmosphärendruck, versperrt.

13. Vorrichtung gemäß Anspruch 12, wobei das Auftriebsmittel gestattet, daß Umgebungsluft durch die erste Säule (40) in die Ansaug-Kontrollkammer eingelassen wird, sobald der durch die Unterdruckquelle (58) erzeugte Unterdruck größer ist als der gewünschte Unterdruck am Patienten.

14. Ansaug-Kontrollkammer gemäß Anspruch 10, wobei das Auftriebsmittel eine schwimmfähige Auftriebskammer (46) enthält und das Ballastmittel (64) eine Ballasthülse (64) umfaßt und wobei sich die Ballasthülse von der Auftriebskammer (46) aus nach oben erstreckt, um beweglich einen Teil der ersten Säule (40) zu umschließen.

15. Ansaug-Kontrollkammer gemäß Anspruch 14, wobei die Ballasthülse (64) einen oberen (66) und einen unteren Abschnitt (68) umfaßt, wobei der untere Abschnitt (68) eine Öffnung umfaßt, durch die eine Fließkommunikation zwischen der ersten und der zweiten Säule (40,42) ermöglicht wird.

16. Vorrichtung gemäß einem der Ansprüche 1 bis 5, wobei die zweite Säule (42) größer ist als die erste Säule (40) und im wesentlichen die erste Säule (40) umgibt.

## Revendications

1. Dispositif de drainage de cage thoracique (10) destiné à extraire des fluides et des gaz du corps d'un patient comprenant :
une chambre de collecte (12) destinée à être en communication d'écoulement avec le corps d'un patient dans laquelle du fluide provenant du corps d'un patient doit être recueilli;
la chambre de collece étant destinée à être en communication avec une source de dépression (58) qui applique une dépression souhaitée au corps d'un patient afin d'aspirer des fluides dans la chambre de collecte;
une chambre de réglage d'aspiration contenant du liquide (16), destinée à être en communication d'écoulement avec l'atmosphère et avec la source de dépression (58);
la chambre de réglage d'aspiration (16) comportant une première et une seconde colonne (40, 42) qui y sont aménagées et la première et la seconde colonne comportant des extrémités supérieures et des extrémités inférieures, les extrémités inférieures étant en communication d'écoulement l'une avec l'autre et l'extrémité supérieure de la première colonne étant en communication d'écoulement avec l'atmosphère, tandis que l'extrémité supérieure de la seconde colonne est en communication d'écoulement avec la source de dépression (58), lorsque le dispositif est en service, caractérisé en ce que la chambre de réglage comprend :
un moyen à flotteur (46) en communication d'écoulement avec la première colonne (40) étant entendu qu'en service, le moyen à flotteur restreint sélectivement l'écoulement de fluide entre la source de dépression (58) et l'atmosphère pour régler le degré de dépression appliqué au corps d'un patient par la source de dépression (58).

2. Dispositif suivant la revendication 1, dans lequel le moyen à flotteur comprend un corps flottant (46) installé activement dans le liquide contenu dans la chambre de réglage d'aspiration (16) et en communication d'écoulement avec l'extrémité inférieure de la première colonne (40).

3. Dispositif suivant la revendication 1 ou 2, dans lequel la chambre de réglage d'aspiration (16) comprend, en outre, un élément amortisseur (80) qui s'étend vers l'intérieur dans la chambre de réglage d'aspiration (16) vers une position adjacente au corps flottant (46) pour former une section de chambre inférieure (84) destinée à empêcher l'écoulement de liquide dans la chambre de réglage d'aspiration (16).

4. Dispositif suivant la revendication 1, 2 ou 3, dans lequel le moyen à flotteur (46) permet de refouler de l'air atmosphérique dans la chambre de réglage d'aspiration (16) à travers la première colonne (40) lorsque la dépression appliquée par la source de dépression (58) est supérieure à la dépression souhaitée pour le patient.

5. Dispositif suivant l'une quelconque des revendications précédentes dans lequel le liquide dans la chambre de réglage d'aspiration (16) forme une hauteur d'eau dynamique entre la première et la seconde colonne (40, 42) pour s'opposer à une rentrée d'air en provenance de l'atmosphère dans la chambre de réglage d'aspiration (16) et le moyen à flotteur (46) exerce une force de flottabilité additive pour s'opposer à une rentrée d'air en provenance de l'atmosphère dans la chambre de réglage d'aspiration (16).

6. Ensemble de chambre de réglage d'aspiration (16) en communication d'écoulement avec une chambre de collecte d'un dispositif de drainage destiné à extraire des fluides du corps d'un patient, dans lequel l'ensemble de chambre de réglage d'aspiration comprend :
une première colonne (40) comportant des extrémités supérieure et inférieure, l'extrémité supérieure étant en communication d'écoulement avec l'atmosphère;
une seconde colonne (42) comportant des extrémités supérieure et inférieure, l'extrémité inférieure étant en communication d'écoulement avec la première colonne et l'extrémité supérieure étant en communication d'écoulement avec une source de dépression (58), caractérisé en ce que l'ensemble comprend :
un élément à flotteur (46) en communication d'écoulement de par sa flottabilité avec la première colonne (40) pour restreindre sélectivement la communication d'écoulement entre la source de dépression (58) et l'atmosphère afin de régler le degré de dépression appliqué au corps d'un patient.

7. Chambre de réglage d'aspiration suivant la revendication 6, dans laquelle la chambre de réglage d'aspiration (16) comprend, en outre, un amortisseur (80) dans l'extrémité inférieure de la chambre de réglage d'aspiration (16) pour y former une chambre limitant l'écoulement du liquide.

8. Dispositif de drainage de cage thoracique suivant l'une quelconque des revendications 1 à 5, caractérisé en ce que la chambre de réglage (16) comprend au moins un élément de lestage (64) qui s'étend vers le haut à partir du moyen à flotteur (46), à l'intérieur de la seconde colonne (42).

9. Dispositif suivant la revendication 8, dans lequel le moyen à flotteur (46) comprend une chambre de flotteur (46) en communication d'écoulement avec l'extrémité inférieure de la première colonne (40).

10. Dispositif suivant la revendication 8 ou 9, dans lequel l'élément de lestage comprend une douille de lestage (64) entourant en substance la première colonne (40).

11. Dispositif suivant la revendication 8, 9 ou 10 dans lequel l'élément de lestage (64) a une densité approximativement égale à la densité du liquide contenu dans la chambre de réglage d'aspiration (16).

12. Dispositif suivant la revendication 11, dans lequel le moyen à flotteur (46) comprend une chambre de flotteur (46) qui bloque sélectivement l'extrémité inférieure de la première colonne (40) en réponse à la différence entre la dépression appliquée par la source de dépression (58) au moyen à flotteur et la pression atmosphérique.

13. Dispositif suivant la revendication 12, dans lequel le moyen à flotteur permet de refouler de l'air atmosphérique dans la chambre de réglage d'aspiration à travers la première colonne (40) lorsque la dépression appliquée par la source de dépression (58) est supérieure à la dépression souhaitée pour le patient.

14. Chambre de réglage d'aspiration suivant la revendication 10, dans lequel le moyen à flotteur comprend une chambre de flotteur (46) et l'élément de lestage (64) comprend une douille de lestage (64), cette douille s'étendant vers le haut à partir de la chambre de flotteur (46) de manière à entourer de façon mobile une partie de la première colonne (40).

15. Chambre de réglage d'aspiration suivant la revendication 14, dans laquelle la douille de lestage (64) comprend une section supérieure (66) et une section inférieure (68), la section inférieure (68) comprenant une ouverture destinée à permettre une communication d'écoulement entre la première et la seconde colonne (40, 42).

16. Dispositif suivant l'une quelconque des revendications 1 à 5, dans lequel la seconde colonne (42) est plus grande que la première colonne (40) et renferme en substance la première colonne (40).
